# EUROPEAN PATENT SPECIFICATION

(11) **EP 1 539 817 B1**
(45) Date of publication and mention of the grant of the patent: **09.11.2011**
(21) Application number: 03795518.4
(22) Date of filing: 27.08.2003
(51) Int. Cl.: C07K 14/81, A61K 38/57, C07K 16/18

(54) **IMMUNOASSAYS FOR SPECIFIC DETERMINATION OF SCCA ISOFORMS**
IMMUNASSAYS ZUR SPEZIFISCHEN BESTIMMUNG VON SCCA-ISOFORMEN
IMMUNO-ESSAIS SERVANT A EFFECTUER LA DETERMINATION SPECIFIQUE D'ISOFORMES DE SCCA

(30) Priority: 10.09.2002 SE 0202702
(43) Date of publication of application: 15.06.2005
(73) Proprietor: Fujirebio Diagnostics AB, 414 55 Göteborg (SE)
(72) Inventor: RÖJER, Eva, S-414 57 Göteborg (SE); OLLE, Nilsson, 417 64 GÖteborg (SE)
(74) Representative: Inger, Lars Ulf Bosson
(86) International application number: PCT/SE2003/001332
(87) International publication number: WO 2004/024767

(56) References cited:
- WO-A1-01/02603
- WO-A1-90/05540
- WO-A2-02/074904
- SULE CATALTEPE ET AL.: 'Development of specific monoclonal antibodies and a sensitive discriminatory immunoassay for the circulating tumor markers SCCA1 and SCCA2' CLINICA CHIMICA ACTA vol. 295, no. 1-2, May 2000, pages 107 - 127, XP002198888
- STENMAN JAKOB ET AL.: 'Relative levels of SCCA2 and SCCA1 mRNA in primary tumors predicts recurrent disease in squamous cell cancer of the head and neck' INT. J. CANCER (PRED. ONCOL.) vol. 95, 2001, pages 39 - 43, XP002974323
- SULE CATALTEPE ET AL.: 'Co-expression of the squamous cell carcinoma antigens 1 and 2 in normal adult human tissues and squamous cell carcinomas' JOURNAL OF HISTOCHEMISTRY AND CYTOCHEMISTRY vol. 48, January 2000, pages 113 - 122, XP002974324
- AKIHIRO MURAKAMI ET AL.: 'Specific detection and quantitation of SCCA antigen 1 and SCC antigen 2 mRNAs by fluorescence-based asymmetric semi-nested reverse transcription PCR' TUMOR. BIOL. vol. 21, 2000, pages 224 - 234, XP002974325

## Description

### FIELD OF THE INVENTION

The present invention relates to the specific determination of different isoforms of SCCA and the use of the serological concentration of the different isoforms and ratio between them as a means of diagnosis of cancer.

### BACKGROUND OF THE INVENTION

Squamous cell carcinoma antigen (SCCA) is a serological marker for squamous cell carcinomas (SCC) of the uterine, cervix, lung, head and neck, vulva, and esophagus (1, 2). It was originally purified in the end 70-ties by Kato and coworkers from the TA-4 complex from human cervical squamous cell carcinoma, with a molecular weight of 42-48 kDa (1, 3). Electrophoresis of the TA-4 complex revealed more than 10 fractions and iso-electric focusing of the antigen suggested two subfractions, an acidic (pI<6.25) and a neutral (pI≥6.25) isoform (4).

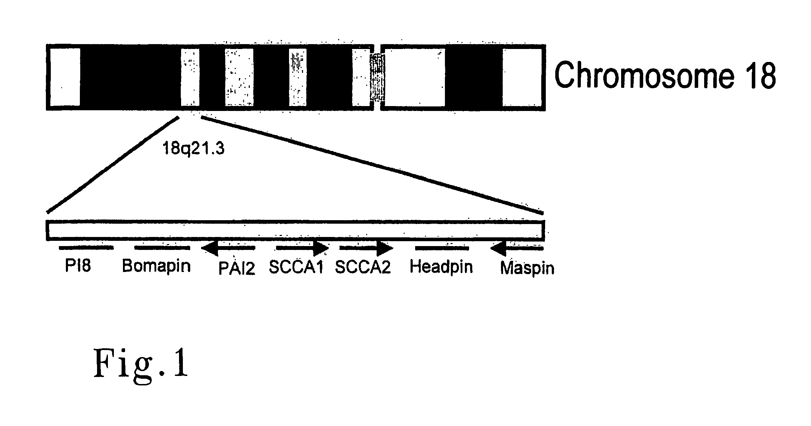

Cloning of the cDNA of *SCCA* shows that it belongs to the family of serine protease inhibitors (serpins) (6). Further cloning of the genomic region on chromosome 18q21.3 revealed two tandemly arrayed genes (7). The more telomeric one, the original *SCCA,* was designated *SCCA1,* whereas the more centromeric one was designated *SCCA2* (Figure 1). They both contain eight exons and the putative intron-exon boundaries, splice sites, initiation codons, and terminal codons are identical. They are 98% identical at the nucleotide level and 92% identical at the amino acid level. The deduced pI values of the SCCA1 and SCCA2 gene products show that the neutral isoform are coded by *SCCA1* and the acidic isoform by *SCCA2*.

In humans the serpins map to one of two chromosomal clusters. *PI6, PI9* and *ELNAH2* map to 6p25, whereas *PI8, Bomapin, PAI2, SCCA1, SCCA2, Headpin* and *Maspin* map to 18q21.3 (Figure 1)(7-12). These clusters are supposed to have arisen via two independent interchromosomal duplications and several rounds of intrachromosomal duplications (9). The chromosome region 18q has often been reported as a region with high frequency of rearrangements (9, 13-16). The targets and functions of serpins are not fully understood. For most, the primary functions are regulation of proteolytic events associated with coagulation, fibrinolysis, apoptosis and inflammation, but alternative functions such as hormone transport and blood pressure regulation have been reported (17-24).

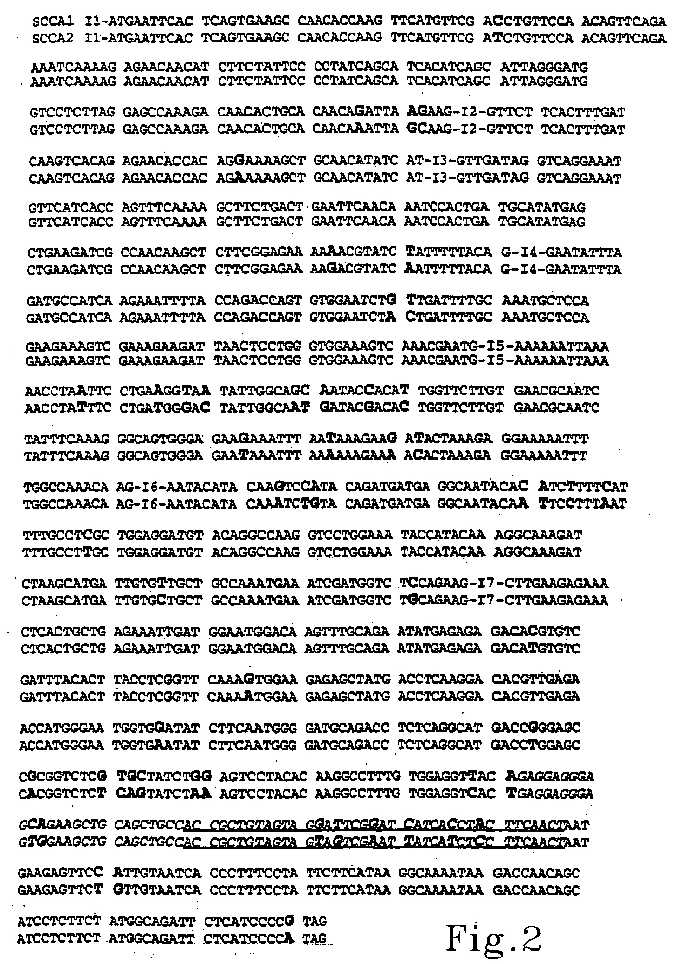

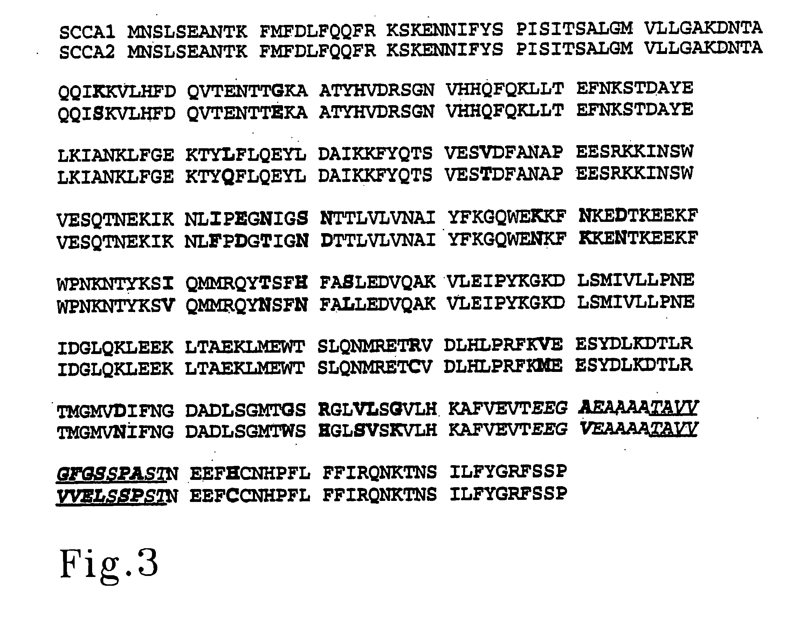

Although SCCA1 and SCCA2 are nearly identical they differ in their reactive site loops (Figure 2 and 3). SCCA1 inhibits the papain-like cystein proteinases cathepsin S, K, and L (25, 26) while SCCA2 inhibits the chymotrypsin-like serine proteinases cathepsin G and mast cell chymase (27). Studies of the reactive site loop (RSL) of SCCA1 show that the RSL is essential for cystein proteinase inhibition (28). The variable portion of the RSL dictates the specificity of the target proteinases shown by RSL swap mutants of SCCA1 and SCCA2 and single mutants (28, 29). It is likely that serpins utilize a common RSL-dependent mechanism to inhibit both serine and cystein proteinases.

The biological role of SCCA1 and SCCA2 are not fully understood. They are considered to be inhibitory serpins. Data suggest that SCCA are involved in apoptosis and expression makes cancer cells resistant to several killing mechanisms by inhibition of apoptosis (30).

SCCA1 and SCCA2 are detected in the cytoplasm of normal squamous epithelial cells (31, 33). The antigen, which appears in the serum of patients, may be a function of SCCA-overproduction by tumor cells and their normal turn over (34). It has been reported that the SCCA detected in serum by using antibody radioimmunology-assay or real-time-PCR, RT-PCR, is mainly SCCA2 (1, 35, 36) but other studies using PCR indicate that both antigens can be amplified and detected in patient samples (37).

Serum concentrations in patients with SCC are correlated to the clinical stage and to the degree of histological differentiation of the tumor (1). For cervical cancer several studies show a correlation between the pretreatment values and the clinical outcome (1, 38-43). Studies also show a correlation between high SCCA levels and tumor volume. Recurrence or progressive disease could be detected several months before clinical evidence (39). Similar results are seen for squamous cell carcinomas of the lung, vulva, head and neck and esophagus (1, 2, 44, 45). In all these studies, they have measured the total SCCA level. For example, Cataltepe et al. (33) discloses the development of specific monoclonal antibodies and a sensitive discriminatory assay for the circulating tumor markers SCCA1 and SCCA2. However, the antibodies according to Cataltepe et al. (33) can only be used for the determination of "total" SCCA1 or SCCA2. They are only useful for the determination of highly elevated levels of these molecules and are thus neither suitable for distinguishing between cancer and healthy subjects, nor for the monitoring of therapy response and detection of recurrent disease.

SCCA's belong to the serpin family and it is likely that different forms of the serpins may be detected in tissue and in circulation. The general function of serpins is to regulate the activity of different proteolytic enzymes, and it may be speculated that also the SCCA1 and SCCA2 in tissues and serum may occur as the "free" serpin and as a complex with their target proteases. This would be similar to the serine protease PSA that in serum mainly is found as a complex with the serpin alfa1-antichymotrypsin. The specific determination of SCCA1 and SCCA2 as well as the respective "free" and complex form of the respective serpin may also provide additional clinical information as compared to "total" SCCA.

### SUMMARY OF THE INVENTION

The present invention discloses the establishment of monoclonal antibodies capable of distinguishing between SCCA1 and SCCA2 as well as between the "free" and "total" amount of the respective serpin. In addition the invention describes the use of the established discriminatory antibodies for the design of immunoassays for determination of the total and "free" form of the SCCA1 and SCCA2 serpins, as well as the use of the immunoassays for diagnosis of cancer and detection of recurrent disease.

### DESCRIPTION OF SPECIFIC EMBODIMENTS

Establishment of monoclonal antibodies against epitopes of SCCA1 and SCCA2, as well as Pan SCCA exposed and hidden in the serine protease complex of the SCCA's, respectively, made it possible to design specific immunoassays for determination of the respective form of SCCA. Furthermore methods for diagnosis of cancer using the specific immunoassays are disclosed within the present invention.

### EXAMPLE 1

### Production of recombinant SCCA

### 1.1 Cloning of SCCA

mRNAs from the cell-lines Caski (cervix), C-41 (cervix), A549 (lung), and RPMI2650 (pharynx) were prepared using QuickPrep Micro mRNA Purification kit (Pharmacia) and cDNA was prepared using First-Strand cDNA Synthesis kit (Pharmacia). A 1218bp DNA fragment covering the coding sequence of SCCA was amplified by PCR in a 100 µl reaction containing 10 mM Tris-HCl pH 8.85, 25 mM KCI, 5 mM (NH₄)₂SO₄, 2 mM MgSO₄ (Boehringer), 0.2mM dNTP (Pharmacia), 10 µM SCCA 1-7F (DNA sequences for all primers are shown in Table 1), 10 µM SCCA 391-3978, 2 µl cDNA and 2.5 U Pwo-polymerase (Boehringer). After denaturing samples for 5 min at 96°C, a total of 30 cycles were performed, each consisting of denaturation for 15 sec at 96°C, annealing for 15 sec at 60°C, and extension for 30 sec at 72°C. The PCR reaction was completed by a final extension for 10 min at 72°C.

### Detection of SCCA1 and SCCA2

Presence of *SCCA1* in PCR products were detected by cleavage with restriction enzyme SacII, resulting in two fragments, 245 and 973 bp, respectively, or by *SCCA1*-specific PCR using the primers SCCA1-7F and SCCA1 323-329B in a standard PCR reaction (75 mM Tris-HCl pH 8.8, 20 mM (NH₄)₂SO₄, 0.01% Tween 20, 2 mM MgCl₂, 0.2 mM dNTP, 10 µM of each primer, template, and 0.025 U/µl reaction Taq Polymerase; after denaturing samples for 5 min at 96°C a total of 30 cycles were performed, each consisting of denaturation for 15 sec at 96°C, annealing for 15 sec at optimal annealing temperature, and extension for 30 sec at 72°C. The PCR reaction was completed by a final extension for 10 min at 72°C.), Ta=50°C, resulting in a 997 bp fragment. Presence of SCCA2 were detected by standard PCR using SCCA 1-7F and a SCCA2-specific primer, SCCA2 357-363B, Ta=60°C, giving a 1090 bp fragment.

### Cloning

PCR-products were cloned using PCR-Script Amp cloning kit (Stratagene). Colony screenings were performed by PCR as described in 1. 2. Plasmid-DNAs were prepared from selected clones containing *SCCA1* or *SCCA2* using Wizard Plus Minipreps DNA Purification System (Promega).

### DNA sequencing

Clones were sequenced using ABI Prism BigDye Terminator Cycle Sequencing (PE Biosystems). Samples were run on an ABI Prism 310.

### Recloning

Selected clones were recloned into the expression vector pGEX-6P-3 (Pharmacia). Fragments were excised from the PCR-Script Amp vector using BamHI and XhoI and ligated into the expression vector in a 10 µl reaction containing 1×OPA, 1 mM ATP, 50 ng cleaved vector, SCCA insert corresponding to a moles-of-ends vector:insert ratio of 1:5-1:8, and 7.5-10 U T4DNAligase (all from Pharmacia). Reaction tubes were incubated at 10°C overnight and inactivated for 10 min at 65°C. 2-4 µl of the reaction was transformed into E.Coli JM109 (46). Plasmid-DNAs from selected clones were then transformed into E.Coli BL21 for protein expression.

### Maintenance of cloned gene

Plasmid-DNA (pGEX-6P-3 containing the SCCA1/A2 fusion gene) in a 10 mM Tris-HCl (pH 8.0) buffer solution is stored in -80°C. For resuming protein expression, plasmid-DNA is transformed into competent *E.coli* BL21 according to Sambrook et al. (p 1.82-1.84 in ref. 46). For preparation of more plasmid-DNA, transformation into *E*. *coli* JM109 is preferred.

### 1.1.2 Protein expression and purification

### Protein Expression

Expression conditions were determined by small-scale preparations. For large scale expression 500 ml cultures of 2xYT and 100 µg of ampicillin/ml were inoculated with 5 ml over-night culture and grown at 37°C. Protein expression was induced at OD₆₀₀=0.5-1.3 by adding IPTG to a final concentration of 0.1 mM.

### Protein Purification

Cells were harvested by centrifugation for 10 min at 2000 g, washed with 50 ml TE pH 8.0, and dissolved in 3 ml TE/g bacterial pellet. Lysozyme was added to a final concentration of 800 µg/g pellet and the mixtures were incubated on ice for 30-60 min and then frozen over night at -70°C. Magnesium chloride and DNase were added to a final concentration of 12 mM and 20 µg/g pellet, respectively. After incubation on ice for 30 min, samples were centrifuged for 30 min at 40000 g. To each supernatant 0.5 ml of 50% Glutathione Sepharose (Pharmacia) was added and incubated for 30 min-2 h at room temperature with gentle agitation. The slurry was washed 5-7 times using 1×PBS. GST-SCCA fusion protein was eluated using 0.5-1 ml Reduced Glutathione (Pharmacia) and incubated for 30-60 min at room temperature or over-night at 4°C, all with gentle agitation. SCCA protein was eluated by cleavage in between GST and SCCA. 0.48 ml cleavage buffer (50 mM Tris-HCI pH 7.0, 150 mM NaCl, 1 mM EDTA, 1 mM DTT) and 20µl PreScission protease were added and samples were incubated at 4°C with gentle agitation for 4 h or over-night. Proteins were analyzed on SDS-PAGE by Phast-system (Pharmacia).

### EXAMPLE 2

### Establishment of hybridomas and monoclonal antibodies

### 2. 1 Immunization and primary selection of Anti SCCA hybridomas

Polyclonal antisera reactive with SCC antigen were obtained by subcutaneous immunization of rabbits with recombinant SCC antigen and collection of immune sera according to standard procedures. The titer of the polyclonal antisera was tested by determination of the reactivity of the antisera with biotinylated SCCA2 and SCCA1 immobilized in streptavidin plates (Labsystems Oy, Helsinki, Finland). The recombinant SCCA2 and SCCA1 were biotinylated with Biotin-N-succinimide caproate ester according to standard procedures.

Monoclonal antibodies reactive with SCCA1 and SCCA2 were obtained by immunization of Balb/c mice intraperitoneally with 10 - 50 µg of recombinant SCCA in Ribi adjuvant. After the immunization and 2 - 4 booster doses during 60 - 90 days spleen cells from the immunized mice were fused with P3 x 63Ag 8 myeloma cells as described.

Hybridomas producing antibodies reacting with SCCA1 and/or SCCA2 were selected by ELISA screening of hybridoma supernatants in microtitre wells coated with affinity purified polyclonal antiserum against mouse IgG + M, (Jackson Immuno Res Lab, US). The wells were then incubated with SCCA antigen, and after washing, the bound antigen was detected by incubation with polyclonal Rabbit Anti SCC and HRP labeled Swine Anti Rabbit Ig (Dako AS, Copenhagen, Denmark).

### 2. 2. Reactivity of selected hybridomas with SCC antigens

The reactivity of the established hybridomas was tested in an ELISA similar to the screening procedure. Briefly the monoclonal antibodies produced by the hybridomas were immobilized in microtitre plates coated with polyclonal antiserum against mouse IgG+M (Jackson Immuno Res Lab, US). The wells were then incubated with 50 µL of the different recombinant SCC antigens (SCCA1, SCCA2, SCCA1/A2 and SCCA2/A1 fusion protein) in PBS 1% BSA for 1 h, after washing the plates were incubated with 100 µL rabbit anti-SCC diluted 1/5000 in PBS-1%BSA and incubated for additional 1h. The bound rabbit Anti-SCC was then detected by incubation with HRP - Swine anti Rabbit Ig and visualized with OPD substrate and determination of OD at 450 nm.

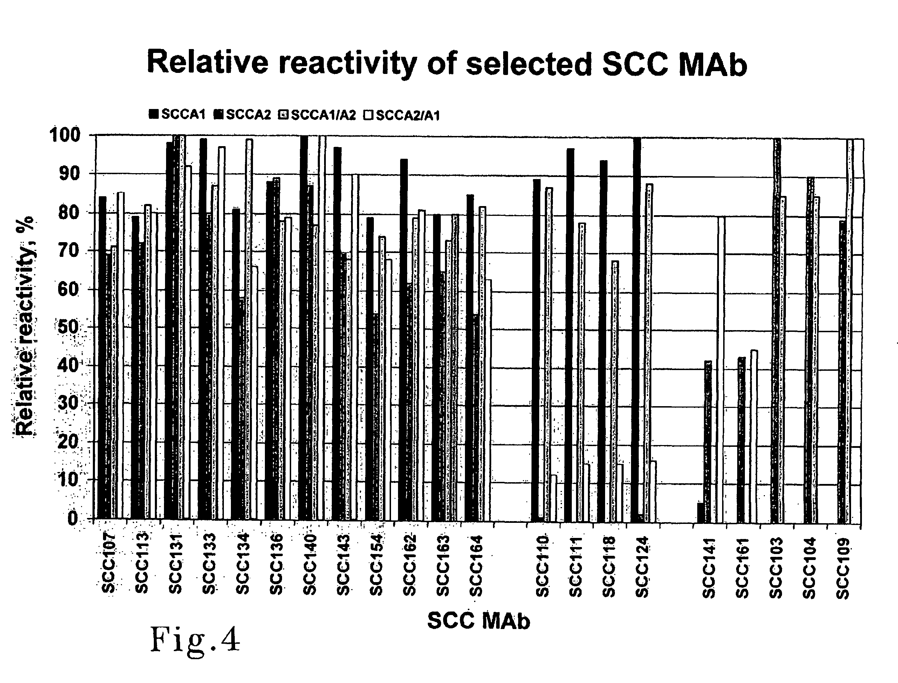

In figure 4 the reactivity of selected hybridomas are shown. They are also evident from the Table 1 below

**Table 1**

| SCC Mab | SCCA1 | SCCA2 | SCCA1/A2 | SCCA2/A1 |
|---|---|---|---|---|
| SCC107 | 84 | 69 | 71 | 100 |
| SCC113 | 79 | 72 | 82 | 100 |
| SCC131 | 98 | 100 | 100 | 92 |
| SCC133 | 99 | 80 | 87 | 97 |
| SCC134 | 81 | 58 | 99 | 66 |
| SCC136 | 88 | 89 | 78 | 79 |
| SCC140 | 100 | 57 | 77 | 100 |
| SCC143 | 97 | 70 | 68 | 90 |
| SCC154 | 79 | 54 | 74 | 68 |
| SCC162 | 94 | 62 | 79 | 81 |
| SCC163 | 80 | 65 | 73 | 80 |
| SCC164 | 85 | 54 | 82 | 63 |
| | | | | |
| SCC110 | 89 | 1 | 87 | 12 |
| SCC111 | 97 | 0 | 78 | 15 |
| SCC118 | 94 | 0 | 68 | 15 |
| SCC124 | 100 | 2 | 88 | 16 |
| | | | | |
| SCC141 | 5 | 42 | 0 | 80 |
| SCC161 | 0 | 43 | 0 | 45 |
| | | | | |
| SCC103 | 0 | 100 | 85 | 0 |
| SCC104 | 0 | 90 | 85 | 0 |
| SCC109 | 0 | 79 | 100 | 0 |

### 2.3 Selection of monoclonal antibodies discriminating between free and complex-bound SCCA

MAb reacting with epitopes exposed in SCCA-protease complexes as well as Mab reacting with epitopes "hidden" in the serpin-protease complex were selected by determination of binding to SCCA-protease complex and to "free" SCCA.

### 2.3.1 Establishment of SCCA-protease complexes

Complex binding of SCCA to target proteases was performed by mixing 2 µg of SCCA-protein with 0.5 µg of Cathepsin G (Biodesign Int.) or 0.5 µg of 0.9 µg Cathepsin L (Calbiochem) in 1×PBS buffer in a total volume of 4.5 µl. Samples were incubated at 37°C for 30 minutes. To each sample, 0.5 µl of 10xComplex-buffer (20% SDS, 140 mM Mercaptoethanol, bromophenolblue) was added. Samples were incubated for 3 minutes at 95°C and analyzed on a 12.5% SDS-PAGE-gel.

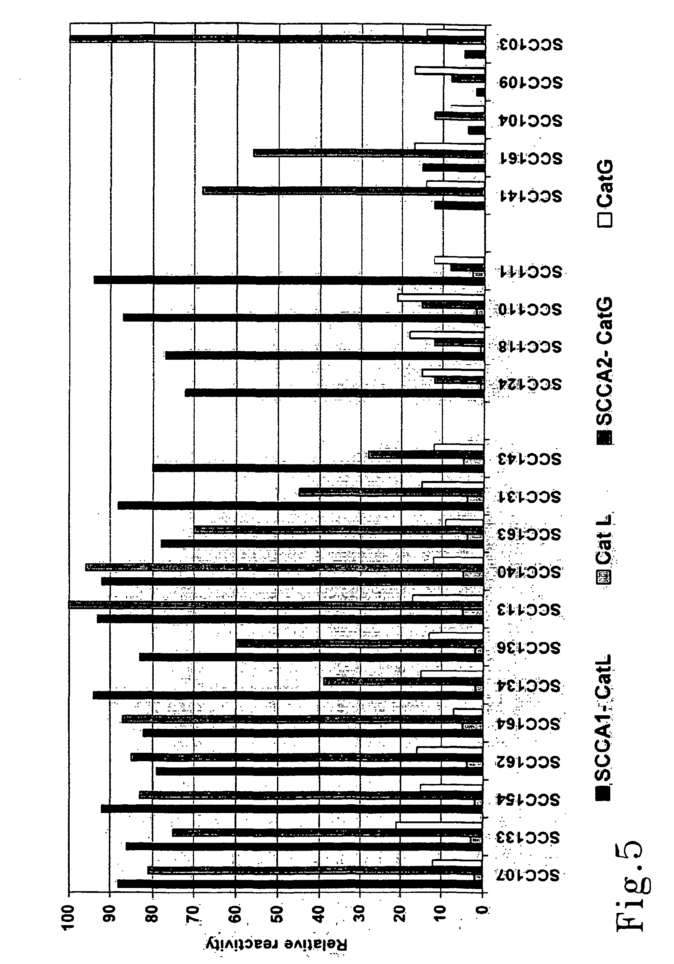

The reactivity of complex binding is evident from the Table 2 below and Figure 5, 2.3.2 Reactivity with SCCA-protease complexes
MAb that recognized epitopes that did not interfere with complex formation between SCCA1 and Cathepsin L and SCCA2 and Cathepsin G, respectively, was detected by preincubation of antibodies recognizing epitopes located within Exon 2 - 7 of SCCA1 and SCCA2 respectively, and then determination of complex formation in ELISA assays as described.

Based on the capability to inhibit the complex formation between SCCA1 and Cathepsin L and SCCA2 and Cathepsin G, respectively it was deduced that a number of antibodies recognized epitopes that were not influenced by the complex formation between the serpins and the target proteases. In figure 5, as well as Table 2 below the reactivity of antibodies with serpin-proteases are shown.

**Table 2**

| SCC Mab | SCCA1- CatL | Cat L | SCCA2- CatG | CatG |
|---|---|---|---|---|
| SCC107 | 88 | 2 | 81 | 12 |
| SCC133 | 86 | 3 | 75 | 21 |
| SCC154 | 92 | 2 | 83 | 15 |
| SCC162 | 79 | 4 | 85 | 16 |
| SCC164 | 82 | 5 | 87 | 7 |
| SCC134 | 94 | 2 | 39 | 15 |
| SCC136 | 83 | 2 | 60 | 13 |
| SCC113 | 93 | 5 | 100 | 17 |
| SCC140 | 92 | 5 | 96 | 12 |
| SCC163 | 78 | 4 | 70 | 9 |
| SCC131 | 88 | 4 | 45 | 15 |
| SCC143 | 80 | 5 | 28 | 12 |
| | | | | |
| SCC124 | 72 | 1 | 12 | 15 |
| SCC118 | 77 | 1 | 12 | 18 |
| SCC110 | 87 | 2 | 15 | 21 |
| SCC111 | 94 | 3 | 8 | 12 |
| | | | | |
| SCC141 | 12 | 0 | 68 | 14 |
| SCC161 | 15 | 0 | 56 | 17 |
| SCC104 | 4 | 0 | 12 | 8 |
| SCC109 | 2 | 0 | 8 | 17 |
| SCC103 | 5 | 0 | 100 | 14 |

The antibodies described in 2.3.1., which reacted with epitopes located in Exon 8 inhibited complex formation between the respective serpin and its protease. It may be deduced that these antibodies recognized "hidden" epitopes.

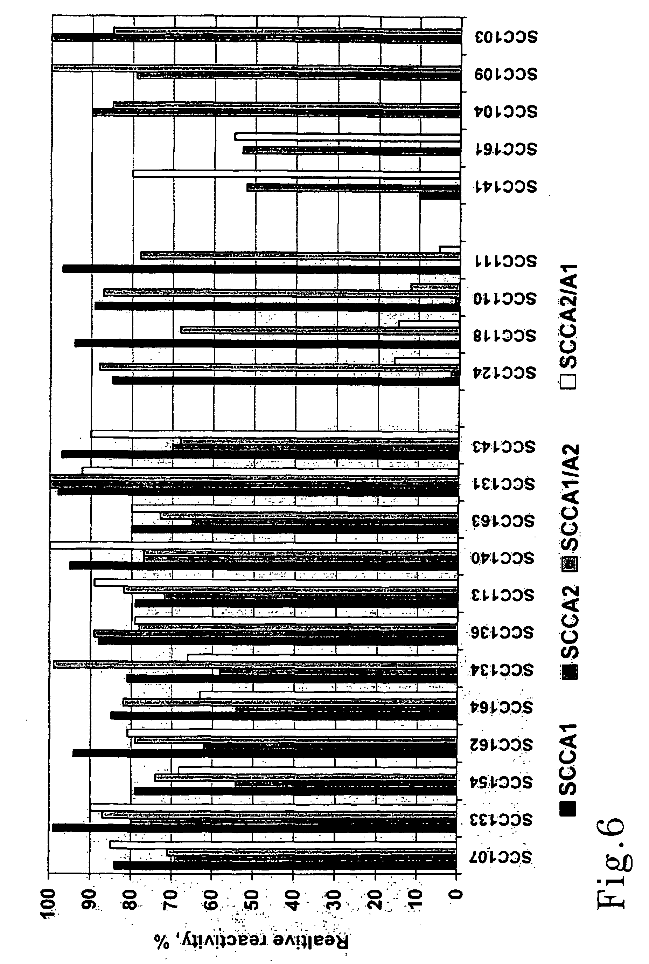

Complexes to "free" SCCA is shown in Table 3 below, as well as inj Figure 6.

**Table 3**

| SCC Mab | SCCA1 | SCCA2 | SCCA1/A2 | SCCA2/A1 |
|---|---|---|---|---|
| SCC107 | 84 | 69 | 71 | 85 |
| SCC133 | 99 | 80 | 87 | 90 |
| SCC154 | 79 | 54 | 74 | 68 |
| SCC162 | 94 | 62 | 79 | 81 |
| SCC164 | 85 | 54 | 82 | 63 |
| SCC134 | 81 | 58 | 99 | 66 |
| SCC136 | 88 | 89 | 78 | 79 |
| SCC113 | 79 | 72 | 82 | 89 |
| SCC140 | 95 | 77 | 77 | 100 |
| SCC163 | 80 | 65 | 73 | 80 |
| SCC131 | 98 | 100 | 100 | 92 |
| SCC143 | 97 | 70 | 68 | 90 |
| | | | | |
| SCC124 | 85 | 2 | 88 | 16 |
| SCC118 | 94 | 0 | 68 | 15 |
| SCC110 | 89 | 1 | 87 | 12 |
| SCC111 | 97 | 0 | 78 | 5 |
| | | | | |
| SCC141 | 10 | 52 | 0 | 80 |
| SCC61 | 0 | 53 | 0 | 55 |
| SCC104 | 0 | 90 | 85 | 0 |
| SCC109 | 0 | 79 | 100 | 0 |
| SCC103 | 0 | 100 | 85 | 0 |

### 2.3.3 Summary of reactivity of established MAb

The reactivity of the established monoclonal antibodies against different forms of SCCA are summarized in Table 4.

**Table 4**

| **Group A PAN SCC MAb** | | | | | **Group B SCCAL MAb** | | **Group C SCCA MAb** | | | |
|---|---|---|---|---|---|---|---|---|---|---|
| **A1a** | **A1b** | **A2** | **A3a** | **A3b** | **B1** | **B2** | **C1a** | **C1b** | **C2a** | **C2b** |
| SCC 107 | SCC 134 | SCC 113 | SCC 140 | SCC 131 | SCC 124 | K134 | SCC 141 | SCC 161 | SCC 103 | SCC 104 |
| SCC 119 | SCC 136 | | SCC 163 | SCC 143 | SCC 118 | K135 | | | | SCC 109 |
| SCC 123 | | | | | SCC 110 | | | | | K122 |
| SCC 128 | | | | | SCC 111 | | | | | |
| SCC 133 | | | | | | | | | | |
| SCC 154 | | | | | | | | | | |
| SCC 162 | | | | | | | | | | |
| SCC 164 | | | | | | | | | | |

| | | | | | | | | | | |
|---|---|---|---|---|---|---|---|---|---|---|
| Groups A1b and A3b react preferentially with "Free" SCC; Groups C1a and C2a recognize "Total SCCA2", while Group C1b and C2b recognize only "Free SCCA2" | | | | | | | | | | |

### 2.4 Production of discriminatory monoclonal antibodies

Monoclonal antibodies were produced by in vitro cultivation of the hybridoma clones by inoculation of 10⁴ cells/mL in DMEM, 5 % Fetal Calf Serum in roller bottles and allowed to grow for 10 - 14 days. The monoclonal antibodies were then purified from the culture medium by Protein A (Bioprocessing Ltd, Durham, UK) affinity chromatography according to the manufacturers recommendation.

### EXAMPLE 3

### Establishment of immunoassays

Using the established monoclonal antibodies and recombinant proteins it was possible to develop immunoassays for specific determination total SCCA and total "free" SCCA, and assays specific for total SCCA1 and "free" SCCA1 as well as assays for specific determination of total SCCA2 and "free" SCCA2, respectively.

### 3. 1. Immunoassays for determination of total SCCA

### 3.1.1 Immunoassays for determination of "total SCCA"

Assays specific for SCCA, i.e the total of "free" SCCA1, "free" SCCA2, complexed SCCA1 and complexed SCCA2 were designed by using antibodies among A1a (Table 1) in combination with antibodies from Groups A2 or A3a.

In the preferred configuration antibody SCC113 was used as catching antibody and SCC107 as detecting antibody.

SCC113 MAb was biotinylated with BiotinNHRS caproate ester, Sigma Chemical Co, US, using standard procedures, and used as catching antibody. SCC107 MAb were conjugated with HRP according to a modification of the Nakone procedure.

The biotinylated SCC113 MAb and HRP conjugated SCC107 MAb were used in one-step EIA according to the following protocol.

### Assay procedure:

1. Add 25 µL of SCCA recombinant antigen (0 - 50 µg/L in PBS, 60 g/L BSA, pH 7.2) + 100 µL of Biotin SCC113 MAb, 1 µg/mL and HRPSCC107, 1 µg/mL in Assay Buffer in Streptavidin coated microtiter plates, Labsystems Oy, Helsinki, Finland.
2. Incubate for 1 h ± 10 min with shaking
3. Wash 6 times with 5 mM Tris buffer, 0.05 % Tween 40, pH 7.75.
4. Add 100 µL TMB, ELISA Technology, US.
5. Incubate 30 min± 5 min
6. Determine OD 620 nm in ELISA reader.

Dose-response curves for free and complex SCCA1 and SCCA2 antigens revealed that the assay recognized all forms of SCCA.
3. 2. Assays for specific determination of SCCA1
3. 2. 1 Assays for total SCCA1

Assays specific for total SCCA1, i.e. Free and Complex SCCA1, without significant reactivity with SCCA2 were designed by using antibodies of Group B1 in combination with antibodies from Group A1a, A2 or A3a. In the preferred configuration SCC110 MAb was used as catching antibody and the SCC107 was used as detecting antibody.

SCC111 MAb was biotinylated with BiotinNHRS caproate ester (Sigma Chemical Co, US) using standard procedures, and used as catching antibody. SCC107 MAb was conjugated with HRP, Type V (Sigma Chemical Co, US), according to a modification of the Nakone procedure.

The biotinylated SCC111 MAb and HRP conjugated SCC107MAb were used in two-site EIA according to the following protocol.

### Assay procedure:

1. Add 50 µL of SCC recombinant antigen (0 - 100 µg/L in PBS, 60 g/L BSA, pH 7.2) + 100 µL of Biotin SCC111 MAb, 2 µg/mL, in Assay Buffer in Streptavidin coated microtiter plates (Labsystems Oy, Helsinki, Finland).
2. Incubate for 1 h ± 10 min with shaking
3. Wash 3 times with 5 mM Tris buffer, 0.05 % Tween 40, pH 7.75.
4. Add 100 µL HRP SCC107 MAb 2 µg/mL, in Assay Buffer.
5. Incubate for 1 h ± 10 min with shaking.
6. Wash 6 times with 5 mM Tris buffer, 0.05 % Tween 40, pH 7.75.
7. Add 100 µL TMB, ELISA Technology, US
8. Incubate 30 min± 5 min
9. Determine OD 620 nm in ELISA reader.

Based on the dose-response curves for SCCA1 and SCCA2 it was concluded that the assay according to example 3.2.1 recognized all forms of SCCA1 with a cross-reactivity of < 5 % for SCCA2.

### 3. 2. 2 Assays for "free" SCCA1

Assays specific for "free" SCCA1, i.e. specific for uncomplexed SCCA1without significant reactivity with complex SCCA1 or SCCA2 were designed by using antibodies of Group B2 in combination with antibodies of Group A1a. In the preferred configuration SCCK134 MAb was used as catching antibody and the SCC107 was used as detecting antibody.

SCCK134 MAb was biotinylated with BiotinNHRS caproate ester (Sigma Chemical Co, US) using standard procedures, and used as catching antibody. SCC107 MAb was conjugated with HRP, Type V (Sigma Chemical Co, US), according to a modification of the Nakone procedure.

The biotinylated SCCK134 MAb and HRP conjugated SCC107 MAb were used in two-site EIA according to the following protocol.

### Assay procedure:

1. Add 50 µL of SCC recombinant antigen (0 - 100 µg/L in PBS, 60 g/L BSA, pH 7.2) + 100 µL of Biotin SCCK134MAb, 2 µg/mL, in Assay Buffer in Streptavidin coated microtitre plates (Labsystems Oy, Helsinki, Finland).
2. Incubate for 1 h ± 10 min with shaking
3. Wash 3 times with 5 mM Tris buffer, 0.05 % Tween 40, pH 7.75.
4. Add 100 µL HRP SCC107MAb 2 µg/mL, in Assay Buffer.
5. Incubate for 1 h ± 10 min with shaking.
6. Wash 6 times with 5 mM Tris buffer, 0.05 % Tween 40, pH 7.75.
7. Add 100 µL TMB, ELISA Technology, US
8. Incubate 30 min± 5 min
9. Determine OD 620 nm in ELISA reader.

Based on the dose-response curves for SCCA1 and SCCA2 it was concluded that the assay according to example 3.2.2 recognized only "FREE" SCCA1 with a cross-reactivity of < 5 % for complex SCCA1 or SCCA2.
3. 3. Assays for specific determination of SCCA2
3. 3. 1 Assays for determination of Total SCCA2

Assays specific for total SCCA2, i.e. free and complex SCCA2, without significant reactivity with SCCA1 were designed by using antibodies of Groups C1a or C2a in combination with antibodies of Group A1a. In the preferred configuration SCC103 MAb was used as catching antibody and the SCC107 was used as detecting antibody.

SCC103 MAb was biotinylated with BiotinNHRS caproate ester (Sigma Chemical Co, US) using standard procedures, and used as catching antibody. SCC107 MAb was conjugated with HRP, Type V (Sigma Chemical Co, US), according to a modification of the Nakone procedure.

The biotinylated SCC103 MAb and HRP conjugated SCC107 MAb were used in two-site EIA according to the following protocol.

### Assay procedure:

1. Add 50 µL of SCC recombinant antigen (0 - 100 µg/L in PBS, 60 g/L BSA, pH 7.2) + 100 µL of Biotin SCC103 MAb, 2 µg/mL, in Assay Buffer in Streptavidin coated microtiter plates (Labsystems Oy, Helsinki, Finland).
2. Incubate for 1 h ± 10 min with shaking
3. Wash 3 times with 5 mM Tris buffer, 0.05 % Tween 40, pH 7.75.
4. Add 100 µL HRP SCC107 MAb 2 µg/mL, in Assay Buffer.
5. Incubate for 1 h ± 10 min with shaking.
6. Wash 6 times with 5 mM Tris buffer, 0.05 % Tween 40, pH 7.75.
7. Add 100 µL TMB, ELISA Technology, US
8. Incubate 30 min± 5 min
9. Determine OD 620 nm in ELISA reader.

Based on the dose-response curves for SCCA1 and SCCA2 it was concluded that the assay according to example 3.3.1 recognized all forms of SCCA2 with a cross-reactivity of < 5 % for SCCA2.

### 3.3.2 Assays for "free" SCCA2

Assays specific for "free" SCCA2, i.e. non-complexed SCCA2, without significant reactivity with SCCA2-protease complex or SCCA1 were designed by using antibodies from Group C2b in combination with antibodies of Group A1a In the preferred configuration SCC104 MAb was used as catching antibody and the SCC107 was used as detecting antibody.

SCC104 MAb was biotinylated with BiotinNHRS caproate ester (Sigma Chemical Co, US) using standard procedures, and used as catching antibody. SCC107 MAb was conjugated with HRP, Type V (Sigma Chemical Co, US), according to a modification of the Nakone procedure.

The biotinylated SCC104 MAb and HRP conjugated SCC107 MAb were used in two-site EIA according to the following protocol.

### Assay procedure:

1. Add 50 µL of SCC recombinant antigen (0 - 100 µg/L in PBS, 60 g/L BSA, pH 7.2) + 100 µL of Biotin SCC104MAb, 2 µg/mL, in Assay Buffer in Streptavidin coated microtiter plates (Labsystems Oy, Helsinki, Finland).
2. Incubate for 1 h ± 10 min with shaking
3. Wash 3 times with 5 mM Tris buffer, 0.05 % Tween 40, pH 7.75.
4. Add 100 µL HRP SCC107 MAb 2 µg/mL, in Assay Buffer.
5. Incubate for 1 h ± 10 min with shaking.
6. Wash 6 times with 5 mM Tris buffer, 0.05 % Tween 40, pH 7.75.
7. Add 100 µL TMB, ELISA Technology, US
8. Incubate 30 min± 5 min
9. OD 620 nm in ELISA reader.

Based on the dose-response curves for SCCA1 and SCCA2 it may be concluded that the immunoassay according to 3.3.2 recognized only "free" SCCA2 with a cross-reactivity of < 5 % for complex SCCA2 or SCCA1

### EXAMPLE 4

### Diagnosis of cancer using immunoassays discriminatory for "free" SCCA.

The immunoassays according to Example 3 were used to determine different forms of SCCA in healthy individuals and in patients with squamous cell carcinoma.
All assays showed discrimination between healthy individuals and cancer patients as expected. However, the discriminatory ratio between healthy and cancer subjects were higher for assays determining SCCA2, which was further improved by determination of the ratio between free and complex SCCA2 and between SCCA2 and SCCA1.

SCCA isoforms were determined in 50 blood donors and in 50 healthy subjects aged 50 - 65 Years in order to determined upper normal level. SCCA isoforms were also determined in the assays according to Example 3 in 94 samples for females diagnosed with cervical cancer and in 20 individuals with squamous cell lung cancer.

### Example 4.1.

The results for Squamous cell lung cancer are shown in Figure 2. SCCA1 was above upper normal level in 14 patients while SCCA2 was elevated in 18 patients. The level of SCCA2 was also relatively higher as compared to SCCA1 and thus improving the discrimination between healthy subjects and individuals with malignant disease

### Example 4.2. SCCA in cervical cancer.

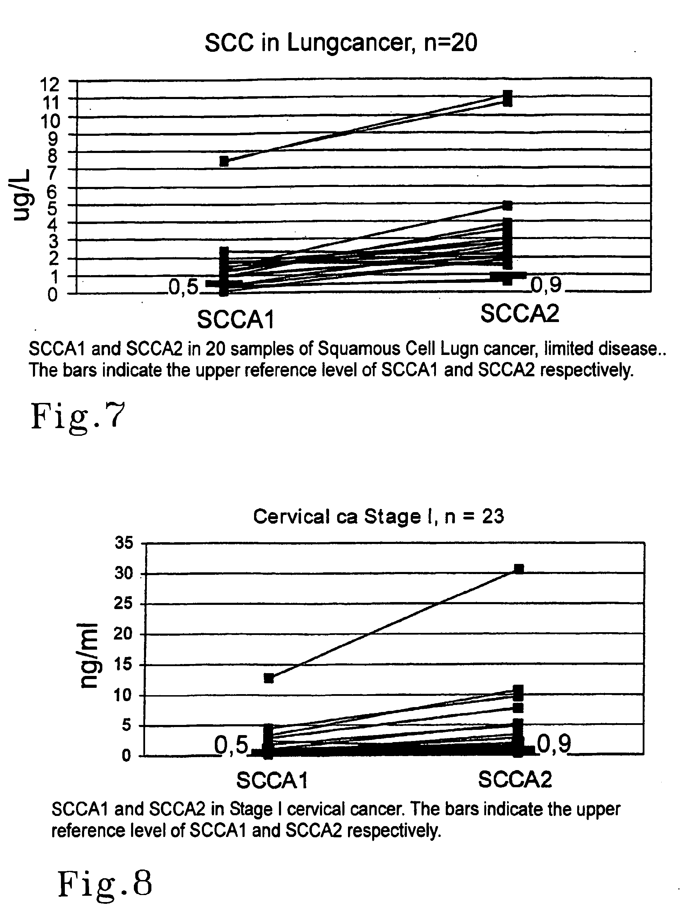

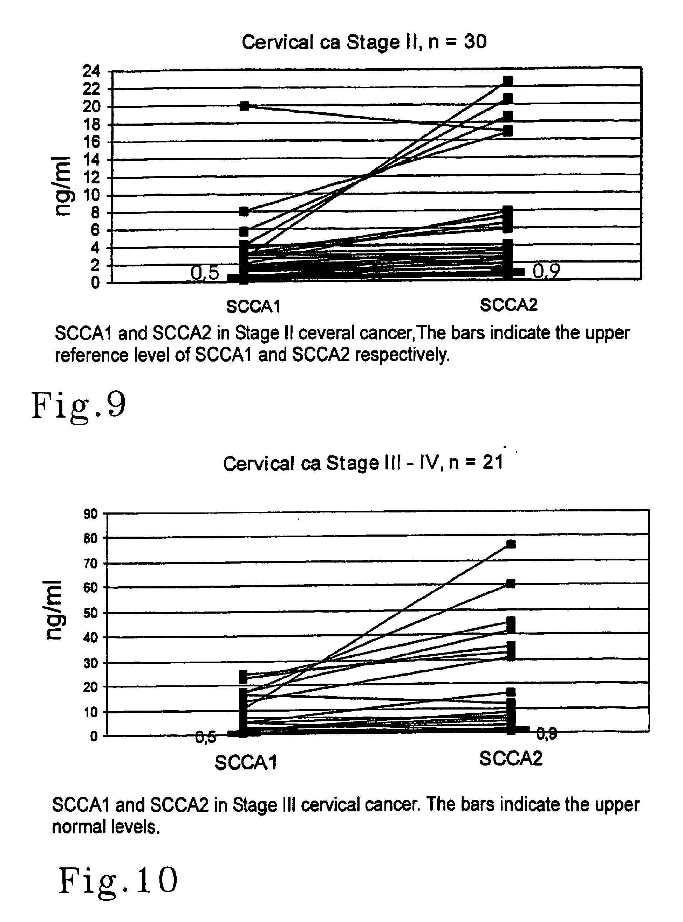

The levels of SCCA1 and SCCA2 in pretherapy samples from females with cervical cancer are shown in Figures 7-10. SCCA2 was in most cases relatively higher elevated as compared to SCCA1. Thus increasing the discrimination between healthy subjects and individuals with cervical cancer.

### Example 4.3 SCCA1 and SCCA2 in therapy monitoring of cervical cancer.

SCCA1 and SCCA2 were determined using assays according to Example 3 in 6 patients during therapy monitoring. Both SCCA1 and SCCA2 followed the clinical course of the disease, and detected recurrent disease prior to clinical manifestation of disease in 4/4 patient. However in the patients the relative increases of SCCA2 was higher compared to SCCA1 thus providing an early signal of recurrent disease. In the patient with NED both SCCA1 and SCCA2 were normalized after the therapy.

Recurrent disease was detected in patient 53 18 months post therapy. The recurrence was indicated by elevated SCCA1 and SCCA2, but SCCA2 responded earlier and showed a higher level as indication of the recurrence as compared to SCCA1.

In patient 29 recurrence was clinically detected 16 months post therapy, which was indicated by elevated SCCA2 from 8 months post therapy, which was 2 - 3 months earlier than SCCA1.

Patient 83 showed progressive disease 7 months post initial therapy. SCCA2 was never normalized, while SCCA1 normalized 3 months after initial therapy and then maws marginally elevated at the time of clinical diagnosis of progressive disease.

Recurrent disease was clinically diagnosed in patient 70 after 13 months. SCCA2 stated to increase between 5 - 6 months post therapy. SCCA1 also was slightly elevated 9 months post therapy and afterwards followed the clinical course. However the SCCA2 more clearly indicated the recurrent disease 5 - 7 months before clinical diagnosis.

SCCA2 levels never normalized in patient 48 suggesting recurrence and progressive disease already 2 months post therapy. SCCA1 was on the upper normal level until 5 months post therapy before increasing.

Patient 45 responded to the treatment and no evidence of disease was noticed after the therapy. This was indicated by both SCCA1 and SCCA2 as the levels were normalized and stayed in the normal range.

Both SCCAL and SCCA2 followed the clinical course of the disease. However SCCA2 provided earlier and more distinct response of recurrent disease as compared to SCCA1.

### Figure legends

FIG. 1 In humans the serpins map to one of two chromosomal clusters. *PI6, PI9* and *ELNAH2* map to 6p25, whereas *P18, Bomapin, PAI2, SCCA1, SCCA2, Headpin* and *Maspin* map to 18q21.3
FIG. 2-3 shows reactive site loops of SCCA1 and SCCA2
FIG. 4 shows relative reactivity of SCC Mabs
FIG. 5 shows relative reactivity of complex bound SCC Mabs
FIG. 6 shows relative reactivity of "free" SCC Mabs
FIG 7 shows SCCA1 and SCCA2 in 20 samples of Squamous Cell Lung cancer, limited disease.
   The bars indicate the upper reference level of SCCA1 and SCCA2 respectively.
FIG. 8. SCCA1 and SCCA2 in Stage I cervical cancer.
   The bars indicate the upper reference level of SCCA1 and SCCA2 respectively.
FIG. 9. SCCA1 ad SCCA2 in Stage II cervical cancer.
   The bars indicate the upper reference level of SCCA1 and SCCA2 respectively.
FIG. 10. SCCA1 and SCCA2 in stage III-IV Cervical cancer.
   The bars indicate the upper normal levels.

### REFERENCES

1. Kato, H. (1992) in Serological Cancer Markers, ed. Sell, S. (The Humana Press, Totowa, NJ), pp. 437-451.
2.Yamawaki, T., Takeshima, N., Shimizu, Y., Teshima, H. & Hasumi, K. (1996) J Obstet Gynaecol Res 22, 341-6.
3. Kato, H. & Torigoe, T. (1977) Cancer 40, 1621-8.
4. Kato, H., Nagaya, T. & Torigoe, T. (1984) Gann 75, 433-5.
5. Suminami, Y., Nawata, S. & Kato, H. (1998) Tumour Biol 19, 488-93.
6. Suminami, Y., Kishi, F., Sekiguchi, K. & Kato, H. (1991) Biochem Biophys Res Commun 181, 51-8.
7. Schneider, S. S., Schick, C., Fish, K. E., Miller, E., Pena, J. C., Treter, S. D., Hui, S. M. & Silverman, G. A. (1995) Proc Natl Acad Sci U S A 92, 3147-51.
8.Bartuski, A. J., Kamachi, Y., Schick, C., Overhauser, J. & Silverman, G. A. (1997) Genomics 43, 321-8.
9. Scott, F. L., Eyre, H. J., Lioumi, M., Ragoussis, J., Irving, J. A., Sutherland, G. A. & Bird, P. I. (1999) Genomics 62, 490-9.
10. Abts, H. F., Welss, T., Mirmohammadsadegh, A., Kohrer, K., Michel, G. & Ruzicka, T. (1999) J Mol Biol 293, 29-39.
11.Spring, P., Nakashima, T., Frederick, M., Henderson, Y. & Clayman, G. (1999) Biochem Biophys Res Commun 264, 299-304.
12. Nakashimaa, T., Pakb, S. C., Silvermanb, G. A., Springa, P. M., Fredericka, M. J. & Claymana, G. L. (2000) Biochim Biophys Acta 1492, 441-446.
13.Silverman, G. A., Bartuski, A. J., Cataltepe, S., Gornstein, E. R., Kamachi, Y., Schick, C. & Uemura, Y. (1998) Tumour Biol 19, 480-7.
14. Katz, S. G., Schneider, S. S., Bartuski, A., Trask, B. J., Massa, H., Overhauser, J., Lalande, M., Lansdorp, P. M. & Silverman, G. A. (1999) Hum Mol Genet 8, 87-92.
15. Gotte, K., Riedel, F., Coy, J. F., Spahn, V. & Hormann, K. (2000) Oral Oncol 36, 360-364.
16. Takebayashi, S., Ogawa, T., Jung, K. Y., Muallem, A., Mineta, H., Fisher, S. G., Grenman, R. & Carey, T. E. (2000) Cancer Res 60, 3397-403.
17. Carrell, R. W. & Evans, D. L. I. (1992) Current Opinion in Structural Biology 2, 438-446.
18. Potempa, J., Korzus, E. & Travis, J. (1994) J Biol Chem 269, 15957-60.
19. Wright, H. T. (1996) Bioessays 18, 453-64.
20. Bird, P. I. (1998) Results Probl Cell Differ 24, 63-89.
21. Bird, C. H., Sutton, V. R., Sun, J., Hirst, C. E., Novak, A., Kumar, S., Trapani, J. A. & Bird, P. I. (1998) Mol Cell Biol 18, 6387-98.
22. Van Patten, S. M., Hanson, E., Bernasconi, R., Zhang, K., Manavalan, P., Cole, E. S., McPherson, J. M. & Edmunds, T. (1999) J Biol Chem 274, 10268-76.
23. Worrall, D. M., Blacque, O. E. & Barnes, R. C. (1999) Biochem Soc Trans 27, 746-50.
24. Sim, R. B. & Laich, A. (2000) Biochem Soc Trans 28, 545-550.
25. Takeda, A., Yamamoto, T., Nakamura, Y., Takahashi, T. & Hibino, T. (1995) FEBS Lett 359, 78-80.
26. Schick, C., Pemberton, P. A., Shi, G. P., Kamachi, Y., Cataltepe, S., Bartuski, A. J., Gornstein, E. R., Bromme, D., Chapman, H. A. & Silverman, G. A. (1998) Biochemistry 37, 5258-66.
27. Schick, C., Kamachi, Y., Bartuski, A. J., Cataltepe, S., Schechter, N. M., Pemberton, P. A. & Silverman, G. A. (1997) J Biol Chem 272, 1849-55.
28. Schick, C., Bromme, D., Bartuski, A. J., Uemura, Y., Schechter, N. M. & Silverman, G. A. (1998) Proc Natl Acad Sci U S A 95, 13465-70.
29. Luke, C., Schick, C., Tsu, C., Whisstock, J. C., Irving, J. A., Bromme, D., Juliano, L., Shi, G. P., Chapman, H. A. & Silverman, G. A. (2000) Biochemistry 39, 7081-91.
30. Suminami, Y., Nagashima, S., Vujanovic, N. L., Hirabayashi, K., Kato, H. & Whiteside, T. L. (2000) Br J Cancer 82, 981-9.
31. Cataltepe, S., Gornstein, E. R., Schick, C., Kamachi, Y., Chatson, K., Fries, J., Silverman, G. A. & Upton, M. P. (2000) J Histochem Cytochem 48, 113-22.
32. Kato, H., Suehiro, Y., Morioka, H., Torigoe, T., Myoga, A., Sekiguchi, K. & Ikeda, I. (1987) Jpn J Cancer Res 78, 1246-50.
33. Cataltepe, S., Schick, C., Luke, C. J., Pak, S. C., Goldfarb, D., Chen, P., Tanasiyevic, M. J., Posner, M. R. & Silverman, G. A. (2000) Clin Chim Acta 295, 107-27.
34. Uemura, Y., Pak, S. C., Luke, C., Cataltepe, S., Tsu, C., Schick, C., Kamachi, Y., Pomeroy, S. L., Perlmutter, D. H. & Silverman, G. A. (2000) Int J Cancer 89, 368-77.
35. Murakami, A., Suminami, Y., Sakaguchi, Y., Nawata, S., Numa, F., Kishi, F. & Kato, H. (2000) Tumour Biol 21, 224-34.
36. Hamakawa, H., Fukizumi, M., Bao, Y., Sumida, T., Onishi, A., Tanioka, H., Sato, H. & Yumoto, E. (1999) Clin Exp Metastasis 17, 593-9.
37. Stenman, J., Lintula, S., Hotakainen, K., Vartiainen, J., Lehvaslaiho, H. & Stenman, U. H. (1997) Int J Cancer 74, 75-80.
38. Crombach, G., Scharl, A., Vierbuchen, M., Wurz, H. & Bolte, A. (1989) Cancer 63, 1337-42.
39. Brioschi, P. A., Bischof, P., Delafosse, C. & Krauer, F. (1991) Int J Cancer 47, 376-9.
40. Duk, J. M., Groenier, K. H., de Bruijn, H. W., Hollema, H., ten Hoor, K. A., van der Zee, A. G. & Aalders, J. G. (1996) J Clin Oncol 14, 111-8.
41. de Bruijn, H. W., Duk, J. M., van der Zee, A. G., Pras, E., Willemse, P. H., Boonstra, H., Hollema, H., Mourits, M. J., de Vries, E. G. & Aalders, J. G. (1998) Tumour Biol 19, 505-16.
42. Tabata, T., Takeshima, N., Tanaka, N., Hirai, Y. & Hasumi, K. (2000) Tumour Biol 21, 375-80.
43.Gaarenstroom, K. N., Kenter, G. G., Bonfrer, J. M., Korse, C. M., Van de Vijver, M. J., Fleuren, G. J. & Trimbos, J. B. (2000) Gynecol Oncol 77, 164-70.
44. Mino, N., Iio, A. & Hamamoto, K. (1988) Cancer 62, 730-4.
45. Snyderman, C. H., D'Amico, F., Wagner, R. & Eibling, D. E. (1995) Arch Otolaryngol Head Neck Surg 121, 1294-7.
46. Sambrook, J., Fritsch, E. F. & Maniatis, T. (1989) *Cold Spring Harbor Laboratory Press, Cold Spring Harbor, NY*.
47. Lindholm, L., Holmgren, J., Svennerholm, L., Fredman, P., Nilsson, O., Persson, B., Myrvold, H. & Lagergard, T. (1983) Int Arch Allergy Appl Immunol 71, 178-81.

## Claims

1. A monoclonal antibody **characterized in that** it selectively binds only free squamous cell carcinoma antigen 2 (SCCA2) molecules, excluding complexed SCCA2 molecules, with a cross-reactivity of <5% for complexed SCCA2 or squamous cell carcinoma antigen 1 (SCCA1) molecules, for use in the detection of squamous cell carcinoma or recurrent squamous cell carcinoma disease.

2. A monoclonal antibody according to claim 1, wherein the antibody has been produced by hybridoma cells, said hybridoma cells deriving from spleen cells from a mammal which have been fused with myeloma cells.

3. An *in vitro* method for detecting SCCA 2 molecules in a sample, **characterized in that**
- monoclonal antibodies according to claims 1 and/or 2 that selectively binds only free SCCA2 molecules are contacted in a sample; and
- the level of binding of said monoclonal antibodies in said sample is compared to the level of binding of the same monoclonal antibodies in a normal sample; and
- an increased level of binding of monoclonal antibodies in the sample compared to the level of binding of the same monoclonal antibodies in the normal sample indicates a presence of squamous cell cancer or recurring squamous cell cancer in the sample.

4. A method according to claim 3, wherein the squamous cell cancer is squamous cell cervical cancer.

5. A method according to claim 3, wherein the squamous cell cancer is squamous cell lung cancer.

6. A method according to claim 3, wherein the squamous cell cancer is squamous cell uterine cancer.

7. A method according to claim 3, wherein the squamous cell cancer is squamous cell esophageal cancer.

8. A method according to claim 3, wherein the squamous cell cancer is squamous cell head and neck cancer.

9. A method according to claim 3, wherein the squamous cell cancer is squamous cell vulva cancer.

10. Use of the monoclonal antibody according to claims 1 and/or 2 for the *in vitro* detection of SCCA2 molecules in a sample.

11. Use according to claim 10, wherein the sample is squamous cell cervical cancer.

12. Use according to claim 10, wherein the sample is squamous cell lung cancer.

13. Use according to claim 10, wherein the sample is squamous cell uterine cancer.

14. Use according to claim 10, wherein the sample is squamous cell esophageal cancer.

15. Use according to claim 10, wherein the sample is squamous cell head and neck cancer.

16. Use according to claim 10, wherein the sample is squamous cell vulva cancer.

17. Use of the monoclonal antibody according to claims 1-2 for the preparation of a diagnostic agent for the *in vitro* detection of SCCA2 molecules in a sample.

## Patentansprüche

1. Monoklonaler Antikörper, **dadurch gekennzeichnet, dass** er selektiv nur freie Plattenepithelkarzinom-Antigen-2- (SCCA2) -Moleküle unter Ausschluss von komplexierten SCCA2-Molekülen bindet, mit einer Kreuzreaktivität von 5% für komplexierte SCCA2- oder Plattenepithelkarzinom-Antigen-1- (SCCA1) -Moleküle, zur Verwendung bei der Erkennung von Plattenepithelkarzinom oder rezidivierender Plattenepithelkarzinomkrankheit.

2. Monoklonaler Antikörper gemäß Anspruch 1, wobei der Antikörper durch Hybridomzellen produziert wurde, wobei die Hybridomzellen von Milzzellen von einem Säugetier stammen, die mit Myelomzellen verschmolzen wurden.

3. In-vitro-Verfahren zum Erfassen von SCCA2-Molekülen in einer Probe, **dadurch gekennzeichnet, dass**
- monoklonale Antikörper gemäß den Ansprüchen 1 und/oder 2, die selektiv nur freie SCCA2-Moleküle binden, in einer Probe kontaktiert werden und
- das Bindungsniveau der monoklonalen Antikörper in der Probe mit dem Bindungsniveau derselben monoklonalen Antikörper in einer Normalprobe verglichen wird und
- ein erhöhtes Bindungsniveau monoklonaler Antikörper in der Probe, verglichen mit dem Bindungsniveau derselben monoklonalen Antikörper in der Normalprobe, ein Vorhandensein von Plattenepithelkrebs oder rezidivierendem Plattenepithelkrebs in der Probe anzeigt.

4. Verfahren gemäß Anspruch 3, wobei der Plattenepithelkrebs Plattenepithel-Zervixkrebs ist.

5. Verfahren gemäß Anspruch 3, wobei der Plattenepithelkrebs Plattenepithel-Lungenkreb ist.

6. Verfahren gemäß Anspruch 3, wobei der Plattenepithelkrebs Plattenepithel-Uteruskrebs ist.

7. Verfahren gemäß Anspruch 3, wobei der Plattenepithelkrebs Plattenepithel-Ösophaguskrebs ist.

8. Verfahren gemäß Anspruch 3, wobei der Plattenepithelkrebs Plattenepithel-Kopfund-Hals-Krebs ist.

9. Verfahren gemäß Anspruch 3, wobei der Plattenepithelkrebs Plattenepithel-Vulvakrebs ist.

10. Verwendung des monoklonalen Antikörpers gemäß den Ansprüchen 1 und/oder 2 für die *In*-*vitro*-Erkennung von SCCA2-Molekülen in einer Probe.

11. Verwendung gemäß Anspruch 10, wobei die Probe Plattenepithel-Zervixkrebs ist.

12. Verwendung gemäß Anspruch 10, wobei die Probe Plattenepithel-Lungenkrebs ist.

13. Verwendung gemäß Anspruch 10, wobei die Probe Plattenepithel-Uteruskrebs ist.

14. Verwendung gemäß Anspruch 10, wobei die Probe Plattenepithel-Ösophaguskrebs ist.

15. Verwendung gemäß Anspruch 10, wobei die Probe Plattenepithel-Kopf-und-Hals-Krebs ist.

16. Verwendung gemäß Anspruch 10, wobei die Probe Plattenepithel-Vulvakrebs ist.

17. Verwendung des monoklonalen Antikörpers gemäß den Ansprüchen 1 - 2 für die Zubereitung eines diagnostischen Mittels für die *In*-*vitro*-Erkennung von SCCA2-Molekülen in einer Probe.

## Revendications

1. Anticorps monoclonal **caractérisé en ce qu'**il se lie sélectivement et uniquement à des molécules d'antigène 2 de carcinome à cellules squameuses (SCCA2) libres, en excluant des molécules SCCA2 complexées, avec une réactivité croisée de < 5 % pour des molécules SCCA2 complexées ou d'antigène 1 de carcinome à cellules squameuses (SCCA1), destiné à être utilisé dans la détection d'une maladie de carcinome à cellules squameuses ou de carcinome à cellules squameuses récidivant.

2. Anticorps monoclonal selon la revendication 1, où l'anticorps a été produit par des cellules d'hybridome, lesdites cellules d'hybridome étant dérivées de cellules de rate d'un mammifère qui ont été fusionnées avec des cellules de myélome.

3. Procédé *in vitro* pour détecter des molécules SCCA2 dans un échantillon, **caractérisé en ce que**
- des anticorps monoclonaux selon les revendications 1 et/ou 2 qui se lient sélectivement et uniquement à des molécules SCCA2 libres sont mis en contact dans un échantillon ; et
- le taux de liaison desdits anticorps monoclonaux dans ledit échantillon est comparé au taux de liaison des mêmes anticorps monoclonaux dans un échantillon normal ; et
- un taux de liaison augmenté des anticorps monoclonaux dans l'échantillon par comparaison au taux de liaison des mêmes anticorps monoclonaux dans l'échantillon normal indique la présence d'un cancer à cellules squameuses ou d'un cancer à cellules squameuses récidivant dans l'échantillon.

4. Procédé selon la revendication 3, dans lequel le cancer à cellules squameuses est un cancer du col de l'utérus à cellules squameuses.

5. Procédé selon la revendication 3, dans lequel le cancer à cellules squameuses est un cancer du poumon à cellules squameuses.

6. Procédé selon la revendication 3, dans lequel le cancer à cellules squameuses est un cancer de l'utérus à cellules squameuses.

7. Procédé selon la revendication 3, dans lequel le cancer à cellules squameuses est un cancer de l'oesophage à cellules squameuses.

8. Procédé selon la revendication 3, dans lequel le cancer à cellules squameuses est un cancer de la tête et du cou à cellules squameuses.

9. Procédé selon la revendication 3, dans lequel le cancer à cellules squameuses est un cancer de la vulve à cellules squameuses.

10. Utilisation de l'anticorps monoclonal selon les revendications 1 et/ou 2 pour la détection *in vitro* de molécules SCCA2 dans un échantillon.

11. Utilisation selon la revendication 10, dans laquelle l'échantillon est un cancer du col de l'utérus à cellules squameuses.

12. Utilisation selon la revendication 10, dans laquelle l'échantillon est un cancer du poumon à cellules squameuses.

13. Utilisation selon la revendication 10, dans laquelle l'échantillon est un cancer de l'utérus à cellules squameuses.

14. Utilisation selon la revendication 10, dans laquelle l'échantillon est un cancer de l'oesophage à cellules squameuses.

15. Utilisation selon la revendication 10, dans laquelle l'échantillon est un cancer de la tête et du cou à cellules squameuses.

16. Utilisation selon la revendication 10, dans laquelle l'échantillon est un cancer de la vulve à cellules squameuses.

17. Utilisation de l'anticorps monoclonal selon les revendications 1-2 pour la préparation d'un agent diagnostique destiné à la détection *in vitro* de molécules SCCA2 dans un échantillon.
